# EUROPEAN PATENT SPECIFICATION

(11) **EP 4 070 747 B1**
(45) Date of publication and mention of the grant of the patent: **25.06.2025**
(21) Application number: 22165620.0
(22) Date of filing: 30.03.2022
(51) Int. Cl.: A61B 17/86, A61B 17/70

(54) **BI-DIRECTIONAL DRILL POINT SCREW**
BIDIREKTIONALE BOHRPUNKTSCHRAUBE
VIS DE POINT DE FORAGE BIDIRECTIONNELLE

(30) Priority: 30.03.2021 US 202163167689 P; 25.03.2022 US 202217656517
(43) Date of publication of application: 12.10.2022
(73) Proprietor: Globus Medical, Inc., Audubon, PA 19403 (US)
(72) Inventor: BONO, Peter L., Bingham Farms, 48025 (US); LARK, James D., West Bloomfield, 48324 (US); SCALES, John S., Ann Arbor, 48105 (US)
(74) Representative: Morabito, Sara

(56) References cited:
- WO-A1-2019/074696
- WO-A1-2019/168663
- US-A1- 2005 222 575
- US-A1- 2011 257 691
- US-A1- 2013 253 517
- US-A1- 2018 368 898

## Description

### FIELD OF THE INVENTION

The present invention relates to an implant for skeletal joint fixation procedures; and more particularly, to a pedicle screw having a bi-directional drill point for oscillating rotational hole formation in a bone.

### BACKGROUND OF THE INVENTION

Joints in the human body often need the bones on opposite sides thereof positionally fixed relative to one another. Such fixation can be needed to correct spinal alignment and to hold replacement joints, such as hip, shoulder, and elbow joints together.

The central nervous system is a vital part of the human physiology that coordinates human activity. It is primarily made up of the brain and the spinal cord. The spinal cord is made up of a bundle of nerve tissue which originates in the brain and branches out to various parts of the body, acting as a conduit to communicate neuronal signals from the brain to the rest of the body, including motor control and sensations. Protecting the spinal cord is the spinal, or vertebral, column. Anatomically, the spinal column is made up of several regions, including the cervical, thoracic, lumbar and sacral regions. The cervical spine is made up of seven vertebrae and functions to support the weight of the head. The thoracic spine is made up of twelve vertebrae and functions to protect the organs located within the chest. Five vertebrae make up the lumbar spine. The lumbar spine contains the largest vertebra and functions as the main weight bearing portion of the spine. Located at the base of the spine are the five fused vertebrae known as the sacrum. The coccyx sits at the base of the spinal column and consists of four fused vertebrae.

Each of the vertebrae associated with the various spinal cord regions are made up of a vertebral body, a posterior arch, and transverse processes. The vertebral body, often described as having a drum-like shape, is designed to bear weight and withstand compression or loading. In between the vertebral bodies is a joint containing an intervertebral disc forming part of a vertebral joint. The intervertebral disc is filled with a soft, gelatinous-like substance which helps cushion the spine against various movements and can be the source of various diseases. The posterior arch of the vertebrae is made up of the lamina, pedicles and facet joints. Transverse processes extend outwardly from the vertebrae and provide the means for muscle and ligament attachment, which aid in movement and stabilization of the vertebrae.

While most people have fully functional spinal cords, it is not uncommon for individuals to suffer some type of spinal ailment, including spondylolisthesis, scoliosis, or spinal fractures. One of the more common disorders associated with the spinal cord is damage to the spinal discs. Damage to the discs results from physical injury, disease, genetic disposition, or as part of the natural aging process. Disc damage often results in intervertebral spacing not being maintained, causing pinching of exiting nerve roots between the discs, resulting in pain. For example, disc herniation is a condition in which the disc material bulges from the disc space between the two vertebrae bodies. It is the bulging of the disc material which causes impingement on the nerves, manifesting in pain to the patient. For most patients, rest and administration of pain and anti-inflammatory medications alleviates the problem. However, in severe cases, cases which have developed into spinal instability or severe disc degeneration, the damaged disc material between the vertebral bodies is removed and replaced with spinal stabilization implants. Restoration to the normal height allows the pressure on the nerve roots to be relieved.

There are many different approaches taken to alleviate or reduce severe spinal disorders. One surgical procedure commonly used is a spinal fusion technique. Several surgical approaches have been developed over the years, and include the Posterior Lumbar Interbody Fusion (PLIF) procedure which utilizes a posterior approach to access the patient's vertebrae or disc space, the Transforaminal Lumbar Interbody Fusion (TLIF) procedure which utilizes a posterior and lateral approach to access the patient's vertebrae or disc space, and the Anterior Lumbar Interbody Fusion (ALIF) which utilizes an anterior approach to access the patient's vertebrae or disc space. Using any of these surgical procedures, the patient undergoes spinal fusion surgery in which two or more vertebrae are linked or fused together through the use of a bone spacing device and/or use of bone grafts. The resulting surgery eliminates any movement between the spinal sections which have been fused together.

In addition to the spinal implants or use of bone grafts, spinal fusion surgery often utilizes spinal instrumentation or surgical hardware, such as pedicle screws, plates, or spinal rods. Once the spinal spacers and/or bone grafts have been inserted, a surgeon places the pedicle screws into a portion of the spinal vertebrae and attaches either rods or plates to the screws as a means for stabilization while the bones fuse. Currently, available systems for inserting the screw into a pedicle can be difficult, particularly in light of the fact that surgeons installing these screws often work in narrow surgical fields. Moreover, since patients can vary with respect to their internal anatomy, resulting in varying and undulating surfaces on the spinal bones, a surgeon may not always have a flat surface or may have anatomical structures that must be maneuvered around in order to properly insert the surgical screws into the pedicle portion of the bone. Difficulty in placing the screws correctly into the pedicle can result in unnecessary increases in the time it takes a surgeon to complete the surgical procedure. Prolonged surgery times increase the risk to the patient. More importantly, improper insertion of the pedicle screw assemblies often results in complications for the patient and requires corrective surgical procedures.

Therefore, there exists a need in the art for a pedicle screw that can be inserted through the bone with oscillating rotation to resist skiving of the screw point across a bone surface when the screw is rotated on an angled surface. The oscillating drill point screw should also allow insertion into rough or undulating surfaces without walking or skiving during penetration of the bone. After the point penetrates the bone, normal rotation of the screw allows the screw to be seated to a normal position. The bone screw should not require full rotation in either direction to penetrate the bone and should cut the bone when oscillated in both directions.

### DESCRIPTION OF THE PRIOR ART

U.S. Patent No. 6,530,929 discloses an installation instrument for placement of a brace or rod into pedicle screws. The instrument is mounted to anchors secured to the pedicle screws utilizing extensions coupled to the anchors. The instrument is movable with respect to the anchors to position a brace in a position more proximate the anchors. The brace can be inserted into the pedicle screws and manipulated away from the installation instrument utilizing a thumb screw. However, a disadvantage associated with the installation instrument for placement of a brace or rod into pedicle screws described therein is that the brace cannot be rotated about its longitudinal axis.

U.S. Patent No. 7,188,626 discloses methods and instruments for placing a brace or connecting element into a plurality of anchors or pedicle screws similar to U.S. Patent No. 6,530,929. Insertion of the connecting elements is accomplished by a linear insertion method, therefore failing to teach a connecting element that can be rotated about its longitudinal axis.

U.S. Patent No. 7,520,879 discloses a device for positioning a connecting element adjacent the spinal column using minimally invasive procedures. An inserter instrument guides the connecting element from a location remote from one or more anchors to a location proximate to the one or more anchors. The extensions are mountable to anchors, and the inserter instrument is mountable to the connecting element for positioning the connecting element adjacent the anchors in a minimally invasive procedure. The inserter instrument does not have to be mounted to the anchors or to the anchor extensions, and is operable independently to position the connecting element into the patient along a minimally invasive insertion path from a location remote from the anchor extensions. While the inserter instrument can rotate the connecting element along its longitudinal axis, it cannot be repositioned on the connecting element to gradually rotate the connecting element in a given direction. Moreover, it cannot be rotated about an axis normal to its longitudinal axis.

U.S. Publication No. 2007/0078460 discloses a method and instrumentation for performing spinal fixation surgery. A first incision is made through the skin, and a passageway is created to the spine. A screw is inserted through the passageway and into a vertebra. The screw has a head portion including a channel. An insertion guide is operably connected to the screw. Additional screws may each be inserted through separate incisions or through the first incision. Insertion guides may be operably connected to a head portion of each screw. A sleeve may be positioned into one insertion guide in a first position to guide a rod through at least one other insertion guide. The sleeve is rotated to a second position to allow the rod to move down the slots of the insertion guides and into the head portion of the screw. Additionally, a holding instrument can be employed to position a rod. Two types of connections between the holding instrument and the rod are described. These connections permit the rotation of the rod about its longitudinal axis, but fail to teach a rod which can be repositioned on the connecting element to gradually rotate the connecting element in a given direction.

U.S. Publication No. 2005/0277934 discloses a minimally invasive spinal fixation system used for spinal arthrodesis or motion preservation spinal repair. The system includes a plurality of pedicle screws, and an attachment assembly for connecting the pedicle screws. The attachment assembly includes a connector for attaching to the first screw and second screw, and a removable guide for percutaneously attaching the connector to the first screw and second screw. The removable guide includes a number of different embodiments for connecting the attachment assembly to the connector. A snap type lock is used to secure the attachment to the connector. While this does permit the connector to be repositioned by rotating it about its longitudinal axis, the repositioning can occur at only 90 degree increments. Moreover, it cannot be rotated about an axis normal to the longitudinal axis of the connector.

US2018/368898 describes a bone screw known in the art.

### SUMMARY

According to the invention it is provided a bi-directional bone screw having the feature of independent claim 1. Further advantageous aspects of the invention are set forth in the dependent claims.

The present invention provides for a bi-directional drill point bone screw. The bi-directional drill point bone screw is constructed and arranged to form a hole by oscillating rotation about the longitudinal axis of the screw. As described herein, the bi-directional drill point bone screw provides a surgeon with a device that can be easily and safely inserted into the bone(s) of a patient to provide fixation of a joint.

As such, the bi-directional drill point bone screw includes at least two, and more preferably three, four or more, flutes each having bi-directional cutting edges which allow the screw to cut and penetrate bone when oscillated or rotated in either direction.

Accordingly, it is a primary objective of the present invention to provide a bi-directional drill point bone screw which is useful in orthopedic surgeries.

It is a further objective of the present invention to provide a bi-directional drill point bone screw that can be rotated in either direction to provide a pilot bore through the bone which allows the screw to be rotated into the bone.

It is yet another objective of the present invention to provide a bi-directional drill point bone screw wherein each flute on the screw tip includes two cutting surfaces, one positioned on each opposite side of the relative flute.

Other objectives and advantages of this invention will become apparent from the following description taken in conjunction with any accompanying drawings wherein are set forth, by way of illustration and example, certain embodiments of this invention. Any drawings contained herein constitute a part of this specification, include exemplary embodiments of the present invention, and illustrate various objects and features thereof.

### BRIEF DESCRIPTION OF THE FIGURES

Figure 1 is a perspective view of one embodiment of the bi-directional drill point bone screw;
Figure 2 is a perspective view of the embodiment shown in Fig. 1, illustrating the bi-directional drill point bone screw secured to an oscillating surgical tool;
Figure 3 is a partial fragmentary view of the bi-directional drill point bone screw as shown in Fig. 2;
Figure 4 is a side view partially in section illustrating the bi-directional drill point bone screw secured to an alternative oscillating tool;
Figure 5 is a side view partially in section illustrating the bi-directional drill point bone screw secured to an alternative oscillating tool;

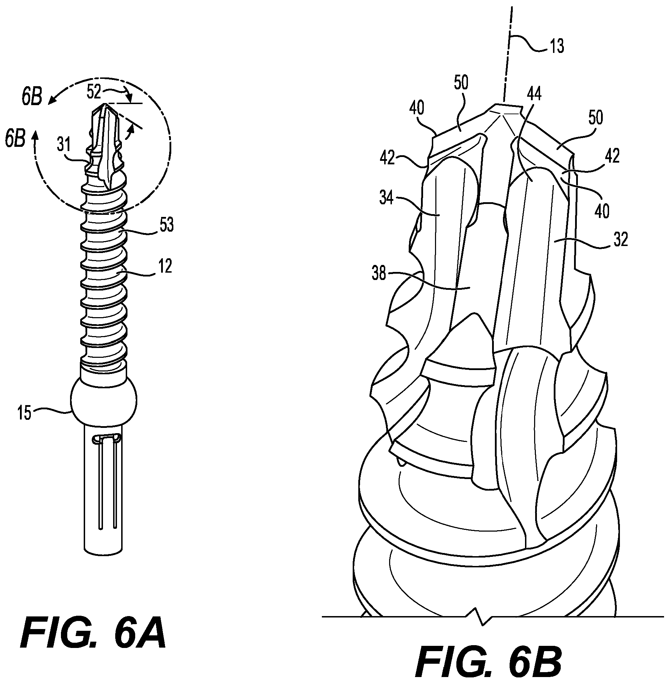

Figure 6A is a perspective view of the bi-directional drill point bone screw;
Figure 6B is a partial enlarged view taken along lines 6B-6B of Figure 6A;

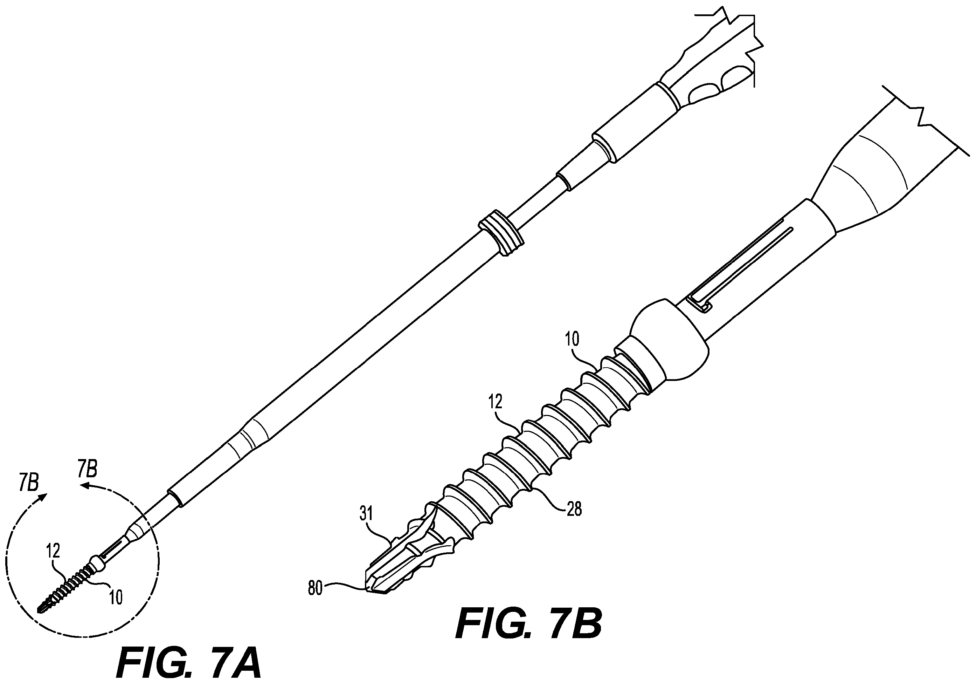

Figure 7A is a perspective view illustrating the bi-directional drill point bone screw in cooperation with a driving tool; and
Figure 7B is a partial enlarged view taken along lines 7B-7B of Figure 7A.

### DETAILED DESCRIPTION

While the present invention is susceptible of embodiment in various forms, there is shown in the drawings and will hereinafter be described presently preferred, albeit not limiting, embodiments with the understanding that the present disclosure is to be considered exemplifications of the present invention and are not intended to limit the invention to the specific embodiments illustrated.

As used herein, "pedicle screw" or "pedicle screw assembly" is used to describe commonly used orthopedic or spinal surgical instrumentation, individually or as units, such as described in U.S. Patent 7,066,937. While many embodiments of a pedicle screw exist commercially, the typical pedicle screw assembly consists generally of the pedicle screw containing a threaded portion which is inserted into a bone or spinal vertebrae. Connected to the screw is a housing unit having upwardly shaped arms which form a U-shape unit, which is often called a "tulip". At the base of the tulip is a saddle that cooperates with both the tulip and the spherical head of the screw to lock the assembly together using a set screw inserted threadably between the two upright elements of the tulip. The housing unit is generally constructed to receive a longitudinal or spinal rod. The longitudinal or spinal rod is set to the housing through use of the set screw, which can be designed to screw into a threaded portion of the housing or tulip to lock the rod into place. This general construction scheme allows the surgeon to connect and secure adjacent bones or bone fragments together through use of the pedicle screw assembly, thereby providing stability temporarily until the bones heal or fuse, or if needed, permanently.

As used herein, the term "proximate end" defines the end closest to the user, i.e., patient, when in use.

As used herein, the "distal end" is defined as the end located farthest from the user when in use.

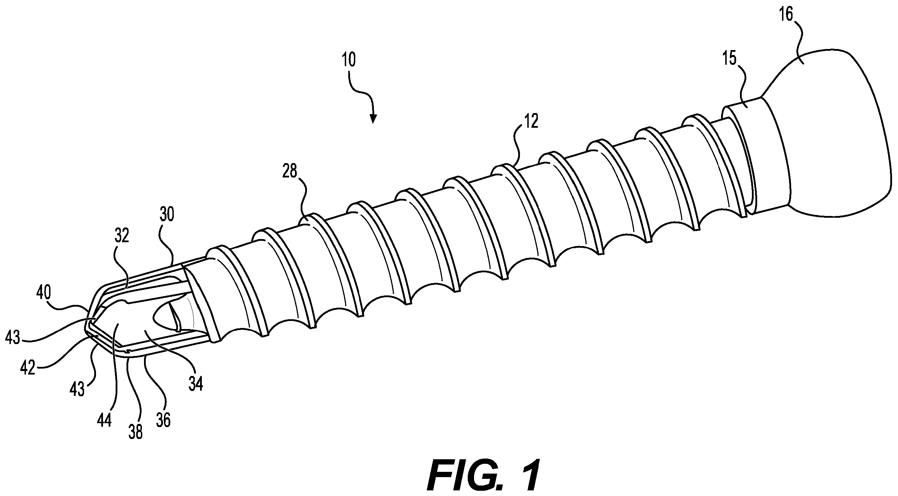

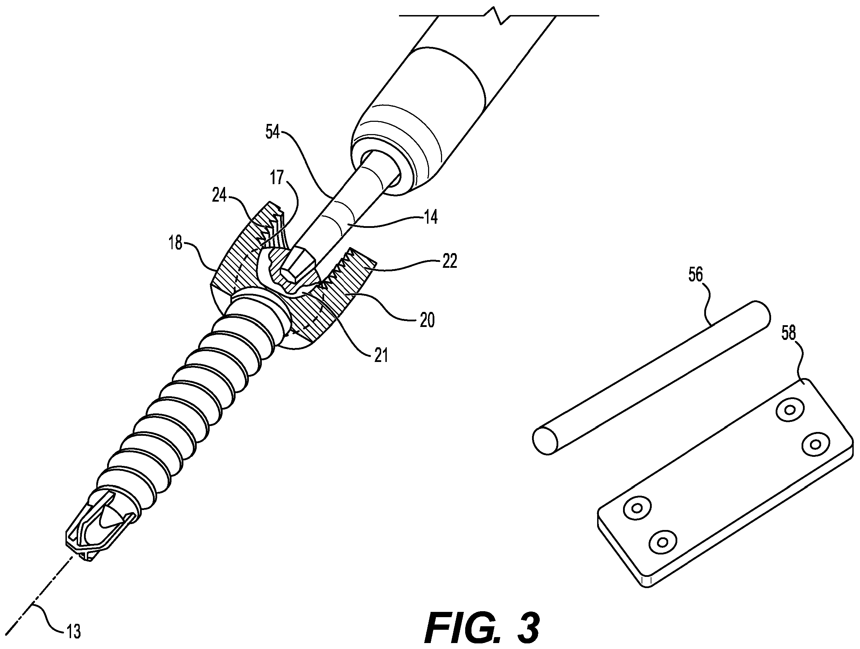

Figures 1-5 illustrate a bi-directional drill point bone screw 10 suitable for use as a portion of a spinal fixation system (not shown). The spinal fixation system may include spinal rods or plates (not shown) connecting a plurality of the oscillating drill point bone screws 10. The present oscillating drill point bone screw 10 may alternatively be utilized in other surgical procedures including, but not limited to, bone fractures, head injuries, or the like. The bi-directional drill point bone screw 10 includes a shank 12 including at least one helical thread 28 extending along at least a portion of an outer surface 53 of the shank that is adapted for insertion into a bone, such as a vertebra. The distal end 15 of the shank 12 has a spherical ball 16, or a portion of a spherical ball, preferably integral therewith, and a tool socket 17 (Fig. 3) therein for the receipt of a tool 14 to install the threaded shank 12 into the bone by rotation thereof. The spherical ball 16 cooperates with a connector 20 to provide a polyaxial connector assembly 18 having a socket 21 receiving the spherical ball 16 therein. The spherical ball 16 and the socket 21 allow the longitudinal axes of the connector assembly 20 and the threaded shank 12, 13 and 54 respectively, to be positioned at angles relative to one another. The connector 20 may also be provided with a pair of opposed channel components 22, which can receive a portion of a rod 56 for securement therein. The rod member is secured in the connector 20 between the two channel components 22, as with a set screw threadably engaging an interior threaded surface 24 of the connector 20. Alternatively, the bi-directional drill point bone screw 10 may be utilized to secure a bone plate 58 across one or more bones to secure the bone(s) in a desired position. The proximal end 31 of the shank 12 includes the bi-directional drill point 30; the bi-directional drill point 30 having at least two, and optionally 3, 4 or more, web components 32 separated by open flutes 34 forming a plurality of webs 36 extending to the proximal tip; each web 36 terminating at the periphery with a land 38. The proximal tip of each web component including a cutting face 50 having at least one, more preferably two or more, cutting edge(s) 40 that is/are constructed and arranged to cut when the screw is rotationally oscillated about the longitudinal axis 13 of the shank 12. Thus, in a preferred embodiment, the cutting face 50 is planar and there is a cutting edge 40 on each side of each flute 34. Each cutting edge 40 preferably includes a face rake 42 which reduces the rotational force required to cut bone. The face rake 42 may be arranged perpendicularly with respect to the cutting face 50 extending along a respective web component 32; or alternatively, the face rake 42 may be arranged to be an angle of less than ninety degrees with respect to the cutting face 50 extending along a respective web component 32. In yet another embodiment, the face rake 42 may be arranged to be an angle of more than ninety degrees with respect to the cutting face extending along a respective web component 32. In at least one embodiment, each cutting face 50 is arranged at an angle with respect to the longitudinal axis 13 to create a point angle 52. The point angle 52 is constructed and arranged to make starting the drill point into a bone easier by not walking across the bone surface when oscillated and reducing the longitudinal pressure required to start an aperture by increasing the force per square inch of surface area. Once the proximal tip 31 of the bi-directional drill point 30 enters the bone, the land 38, which may also include cutting tips, size the hole formed in the bone. In some embodiments, the land 38 may be constructed and arranged to burnish the bone surface as it is sized to create a precision diameter and a smooth bore. In some embodiments, the burnishing may include compression of the cut bone surface. The flutes 34 are sized and shaped to channel away bone fragments and shavings as they are cut. In a preferred embodiment, the flutes 34 include a radiused root 44 that adds strength and rigidity to the bi-directional drill point 30, while still channeling the chips and shavings out of the hole that's being formed.

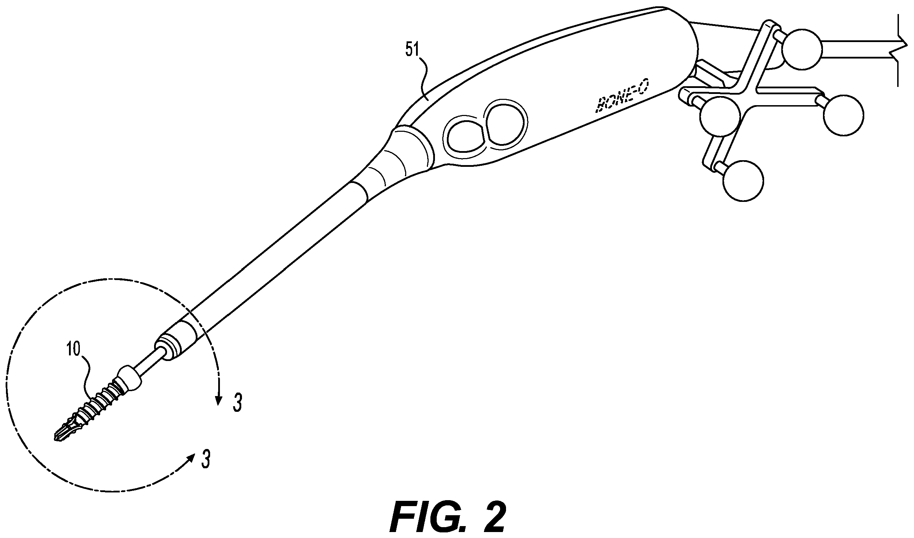

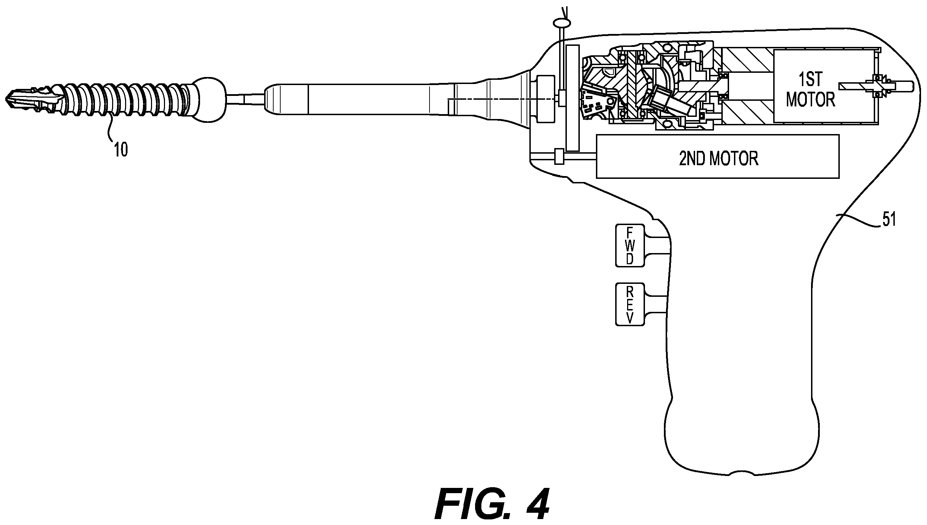

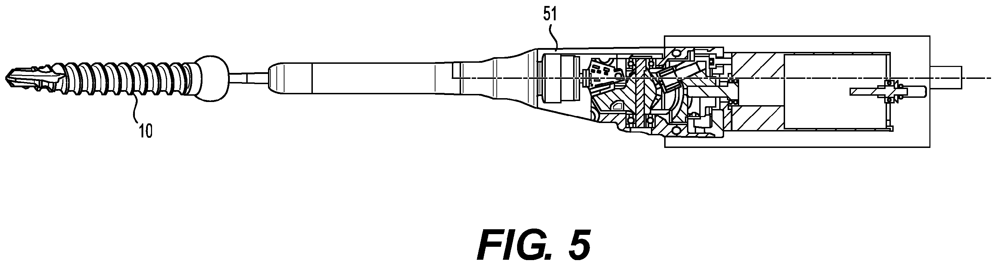

Referring to Figs. 2-5, the bi-directional drill point bone screw 10 is illustrated secured to a surgical tool 51 that is constructed and arranged to oscillate the screw shank about its longitudinal axis 13 until the bi-directional drill point 30 penetrates the bone, and thereafter rotate the screw 10 into its final position in a single direction. Removal of the screw 10 is completed by rotating the screw 10 in an opposite direction with a driving tool 14. Figs. 4 and 5 illustrate alternative devices for oscillating the screw 10, and thereafter providing standard rotation for placing the screw 10 in its final position.

All patents and publications mentioned in this specification are indicative of the levels of those skilled in the art to which the invention pertains.

It is to be understood that while certain forms of the invention are illustrated, it is not to be limited to the specific form or arrangement herein described and shown. It will be apparent to those skilled in the art that various changes may be made without departing from the scope of the invention and the invention is not to be considered limited to what is shown and described in the specification and any drawings/figures included herein.

One skilled in the art will readily appreciate that the present invention is well adapted to carry out the objectives and obtain the ends and advantages mentioned, as well as those inherent therein. The embodiments procedures and techniques described herein are presently representative of the preferred embodiments, are intended to be exemplary, and are not intended as limitations on the scope. Changes therein and other uses will occur to those skilled in the art which are encompassed within the scope of the invention which is defined by appended claims. Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. A bi-directional drill point bone screw (10) comprising;
- a shank (12) including a central longitudinal axis (13), a proximal end (31) and a distal end (15), at least one helical thread (28) extending around and along a portion of an outer surface (53) of the shank (12),
- the at least one helical thread (28) adapted for interlocking cooperation with a bone,
- the proximal end (31) of the shank (12) including a bi-directional drill point (30),
- the bi-directional drill point (30) having at least two web components (32) separated by open flutes (34),
- each web component (32) including a cutting face (50) at the most proximal end of the shank (12), **characterized in that** each cutting face (50) includes at least two cutting edges (40) arranged to rotate around the central longitudinal axis (13) and arranged to cut when the screw is rotationally oscillated about the longitudinal axis (13) of the shank (12),
- wherein at least one cutting edge (40) of the two cutting edges (40) is arranged to cut when the bi-directional drill point bone screw (10) is rotated in a clockwise direction,
- and at least another cutting edge (40) of the two cutting edges (40) is arranged to cut when the bi-directional drill point bone screw (10) is rotated in a counter-clockwise direction, wherein rotary oscillation of the bi-directional drill point bone screw (10) about the longitudinal axis (13) of the shank (12) is suitable to form a predetermined diameter aperture in a bone.

2. The bi-directional drill point bone screw (10) as claimed in Claim 1 wherein the cutting face (50) is planar and there is a cutting edge (40) on each side of each flute (34).

3. The bi-directional drill point bone screw (10) as claimed in Claim 1 wherein each cutting edge (40) includes a face rake (42), the face rake (42) arranged perpendicularly with respect to the cutting face (50) extending along a respective web component (32), and wherein preferably each cutting edge (40) includes a face rake (42), the face rake (42) arranged to be at an angle of less than ninety degrees with respect to the cutting face (50) extending along a respective web component (32), wherein preferably each cutting edge (40) includes a face rake (42), the face rake (42) arranged to be at an angle of more than ninety degrees with respect to the cutting face (50) extending along a respective web component (32).

4. The bi-directional drill point bone screw (10) as claimed in any one of the preceding claims, wherein the bi-directional drill point includes at least three web components (32) separated by open flutes (34), each web component including a cutting face (50) at the most proximal end of the shank (12), each cutting face (50) including at least one cutting edge (40) arranged to rotate around the central longitudinal axis (13), wherein at least one cutting edge (40) is arranged to cut when the bi-directional drill point bone screw (10) is rotated in a clockwise direction and at least one cutting edge (40) is arranged to cut when the bi-directional drill point bone screw (10) is rotated in a counter-clockwise direction.

5. The bi-directional drill point bone screw (10) as claimed in any one of the preceding claims, wherein the bi-directional drill point includes four or more web components (32) separated by open flutes (34), each web component (32) including a cutting face (50) at the most proximal end of the shank (12), each cutting face (50) including at least one cutting edge (40) arranged to rotate around the central longitudinal axis (13), wherein at least two cutting edges (40) are arranged to cut when the bi-directional drill point bone screw (10) is rotated in a clockwise direction and at least two cutting edges (40) are arranged to cut when the bi-directional drill point bone screw (10) is rotated in a counter-clockwise direction.

6. The bi-directional drill point bone screw (10) as claimed in any one of the preceding claims, wherein each web component (32) comprises a radiused root (44) and a web (36), each web (36) terminating at the outer periphery with a land (38), the land (38) constructed and arranged to control the diameter of the aperture created by the bi-directional drill point bone screw (10).

7. The bi-directional drill point bone screw (10) as claimed in Claim 6 wherein the land (38) is constructed and arranged to burnish the bone surface as the aperture is sized, and wherein preferably the land (38) is constructed and arranged to compress the bone surface as the aperture is sized.

8. The bi-directional drill point bone screw (10) as claimed in any one of the preceding claims, wherein each cutting face (50) is a planar surface.

9. The bi-directional drill point bone screw (10) as claimed in Claim 8 wherein each cutting face (50) is arranged at an angle with respect to the longitudinal axis (13) to create a point angle (52), the point angle (52) reducing the longitudinal pressure required to start an aperture.

10. The bi-directional drill point bone screw (10) as claimed in any one of the preceding claims, wherein the flutes (34) are sized and shaped to channel bone fragments and shavings away from the proximal end (31) as the bi-directional drill point bone screw (10) is oscillated.

11. The bi-directional drill point bone screw (10) as claimed in any one of the preceding claims, wherein the at least one helical thread (28) is adapted to cut threads in the aperture as the bi-directional drill point bone screw (10) is rotated into the aperture and wherein preferably the at least one helical thread (28) is adapted to compression form threads in the aperture as the bi-directional drill point bone screw (10) is rotated into the aperture.

12. The bi-directional drill point bone screw (10) as claimed in any one of the preceding claims, wherein the distal end (15) of the shank (12) includes a tool socket (17) for cooperation with a driving tool (14) for rotation and/or oscillation of the shank (12), and wherein preferably the distal end (15) of the shank (12) includes a portion of a spherical ball (16), the portion of the spherical ball (16) formed integral with the shank (12).

13. The bi-directional drill point bone screw (10) as claimed in Claim 12 including a polyaxial connector assembly (18), the polyaxial connector assembly (18) including a socket (21) for receiving the portion of a spherical ball (16) therein, the portion of the spherical ball (16) and the polyaxial connector assembly (18) cooperating to allow the connector longitudinal axis (54) and the shank (12) to be positioned at angles relative to one another.

14. The bi-directional drill point bone screw (10) as claimed in Claim 13 wherein the polyaxial connector assembly (18) is constructed and arranged to cooperate with a rod member (56) for securing a first bi-directional drill point bone screw (10) to a second bi-directional drill point bone screw (10).

15. The bi-directional drill point bone screw (10) as claimed in Claim 13 wherein the polyaxial connector assembly (18) is constructed and arranged to cooperate with a plate member (58) for securing a first bi-directional drill point bone screw (10) to a second bi-directional drill point bone screw (10).

## Patentansprüche

1. Knochenschraube (10) mit bidirektionaler Bohrspitze, umfassend:
- einen Schaft (12) mit einer zentralen Längsachse (13), einem proximalen Ende (31) und einem distalen Ende (15), wobei sich zumindest ein Schraubengewinde (28) um und entlang eines Abschnitts einer Außenfläche (53) des Schafts (12) erstreckt,
- wobei zumindest ein Schraubengewinde (28) zum formschlüssigen Zusammenwirken mit einem Knochen angepasst ist,
- wobei das proximale Ende (31) des Schaftes (12) eine bidirektionale Bohrspitze (30) umfasst,
- wobei die bidirektionale Bohrspitze (30) zumindest zwei Stegkomponenten (32) aufweist, die durch offene Nuten (34) getrennt sind,
- wobei jede Stegkomponente (32) eine Schneidfläche (50) am proximalsten Ende des Schafts (12) umfasst, **dadurch gekennzeichnet, dass** jede Schneidfläche (50) zumindest zwei Schneidkanten (40) umfasst, die angeordnet sind, um sich um die zentrale Längsachse (13) zu drehen und angeordnet sind, um sie zu schneiden, wenn die Schraube drehend um die Längsachse (13) des Schafts (12) hin- und herbewegt wird,
- wobei zumindest eine Schneidkante (40) der zwei Schneidkanten (40) angeordnet ist, um sie zu schneiden, wenn die Knochenschraube (10) mit bidirektionaler Bohrspitze im Uhrzeigersinn gedreht wird,
- und zumindest eine andere Schneidkante (40) der zwei Schneidkanten (40) angeordnet ist, um sie zu schneiden, wenn die Knochenschraube (10) mit bidirektionaler Bohrspitze gegen den Uhrzeigersinn gedreht wird, wobei eine Drehschwingung der Knochenschraube (10) mit bidirektionaler Bohrspitze um die Längsachse (13) des Schafts (12) angepasst ist, um eine Öffnung mit vorbestimmtem Durchmesser in einem Knochen zu bilden.

2. Bidirektionale Knochenschraube (10) mit Bohrspitze nach Anspruch 1, wobei die Schneidfläche (50) eben ist und sich auf jeder Seite jeder Nut (34) eine Schneidkante (40) befindet.

3. Bidirektionale Knochenschraube (10) mit Bohrspitze nach Anspruch 1, wobei jede Schneidkante (40) einen Spanwinkel (42) aufweist, wobei sich der Spanwinkel (42), der senkrecht zur Schneidfläche (50) angeordnet ist, entlang einer jeweiligen Stegkomponente (32) erstreckt, und wobei vorzugsweise jede Schneidkante (40) einen Spanwinkel (42) umfasst, wobei sich der Spanwinkel (42), der angeordnet ist, um in einem Winkel von weniger als 90 Grad zur Schneidfläche (50) zu verlaufen, entlang einer jeweiligen Stegkomponente (32) erstreckt, wobei vorzugsweise jede Schneidkante (40) einen Spanwinkel (42) umfasst, wobei sich der Spanwinkel (42), der angeordnet ist, um in einem Winkel von mehr als 90 Grad zur Schneidfläche (50) zu verlaufen, entlang einer jeweiligen Stegkomponente (32) erstreckt.

4. Bidirektionale Knochenschraube (10) mit Bohrspitze nach einem der vorhergehenden Ansprüche, wobei die bidirektionale Bohrspitze zumindest drei Stegkomponenten (32) umfasst, die durch offene Nuten (34) getrennt sind, wobei jede Stegkomponente eine Schneidfläche (50) am proximalsten Ende des Schafts (12) umfasst, wobei jede Schneidfläche (50) zumindest eine Schneidkante (40) umfasst, die angeordnet ist, um sich um die zentrale Längsachse (13) zu drehen, wobei zumindest eine Schneidkante (40) angeordnet ist, um sie zu schneiden, wenn die bidirektionale Knochenschraube (10) mit Bohrspitze im Uhrzeigersinn gedreht wird, und zumindest eine Schneidkante (40) angeordnet ist, um sie zu schneiden, wenn die bidirektionale Knochenschraube (10) mit Bohrspitze gegen den Uhrzeigersinn gedreht wird.

5. Bidirektionale Knochenschraube (10) mit Bohrspitze nach einem der vorhergehenden Ansprüche, wobei die bidirektionale Bohrspitze vier oder mehr Stegkomponenten (32) umfasst, die durch offene Nuten (34) getrennt sind, wobei jede Stegkomponente (32) eine Schneidfläche (50) am proximalsten Ende des Schafts (12) umfasst, wobei jede Schneidfläche (50) zumindest eine Schneidkante (40) umfasst, die angeordnet ist, um sich um die zentrale Längsachse (13) zu drehen, wobei zumindest zwei Schneidkanten (40) angeordnet sind, um sie zu schneiden, wenn die bidirektionale Knochenschraube (10) mit Bohrspitze im Uhrzeigersinn gedreht wird, und zumindest zwei Schneidkanten (40) angeordnet sind, um sie schneiden, wenn die bidirektionale Knochenschraube (10) mit Bohrspitze gegen den Uhrzeigersinn gedreht wird.

6. Bidirektionale Knochenschraube (10) mit Bohrspitze nach einem der vorhergehenden Ansprüche, wobei jede Stegkomponente (32) eine abgerundete Wurzel (44) und einen Steg (36) umfasst, wobei jeder Steg (36) an der Außenperipherie mit einem Steg (38) endet, wobei der Steg (38) konstruiert und angeordnet ist, um den Durchmesser zu steuern, der durch die bidirektionale Knochenschraube (10) mit Bohrspitze erzeugt wird.

7. Bidirektionale Knochenschraube (10) mit Bohrspitze nach Anspruch 6, wobei der Steg (38) konstruiert und angeordnet ist, um die Knochenoberfläche zu glätten, wenn die Öffnung dimensioniert wird, und wobei der Steg (38) vorzugsweise konstruiert und angeordnet ist, um die Knochenoberfläche zu komprimieren, wenn die Öffnung dimensioniert wird.

8. Bidirektionale Knochenschraube (10) mit Bohrspitze nach einem der vorhergehenden Ansprüche, wobei jede Schneidfläche (50) eine ebene Fläche ist.

9. Bidirektionale Knochenschraube (10) mit Bohrspitze nach Anspruch 8, wobei jede Schneidfläche (50) in einem Winkel zur Längsachse (13) angeordnet ist, um einen Spitzenwinkel (52) zu bilden, wobei der Spitzenwinkel (52) den Längsdruck reduziert, der erforderlich ist, um eine Öffnung zu beginnen.

10. Bidirektionale Knochenschraube (10) mit Bohrspitze nach einem der vorhergehenden Ansprüche, wobei die Nuten (34) dimensioniert und geformt sind, um Knochenfragmente und -späne vom proximalen Ende (31) wegzuleiten, wenn die bidirektionale Knochenschraube (10) mit Bohrspitze hin- und herbewegt wird.

11. Bidirektionale Knochenschraube (10) mit Bohrspitze nach einem der vorhergehenden Ansprüche, wobei das zumindest eine Schraubengewinde (28) angepasst ist, um Gewindegänge in die Öffnung zu schneiden, wenn die bidirektionale Knochenschraube (10) mit Bohrspitze in die Öffnung gedreht wird, und wobei vorzugsweise das zumindest eine Schraubengewinde (28) angepasst ist, um Gewindegänge in der Öffnung durch Kompression zu formen, wenn die bidirektionale Knochenschraube (10) mit Bohrspitze in die Öffnung gedreht wird.

12. Bidirektionale Knochenschraube (10) mit Bohrspitze nach einem der vorhergehenden Ansprüche, wobei das distale Ende (15) des Schafts (12) eine Werkzeugaufnahme (17) zum Zusammenwirken mit einem Antriebswerkzeug (14) zum Drehen und/oder Schwingen des Schafts (12) umfasst, und wobei das distale Ende (15) des Schafts (12) vorzugsweise einen Abschnitt einer Kugel (16) umfasst, wobei der Abschnitt der Kugel (16) einstückig mit dem Schaft (12) ausgebildet ist.

13. Bidirektionale Knochenschraube (10) mit Bohrspitze nach Anspruch 12, die eine polyaxiale Verbindungsanordnung (18) umfasst, wobei die polyaxiale Verbindungsanordnung (18) eine Aufnahme (21) zum Aufnehmen des Abschnitts einer Kugel (16) darin umfasst, wobei der Abschnitt der Kugel (16) und die polyaxiale Verbindungsanordnung (18) zusammenwirken, um eine Positionierung der Verbindungslängsachse (54) und des Schafts (12) in Winkeln relativ zueinander zu ermöglichen.

14. Bidirektionale Knochenschraube mit Bohrspitze (10) nach Anspruch 13, wobei die polyaxiale Verbindungsanordnung (18) konstruiert und angeordnet ist, um mit einem Stabelement (56) zum Befestigen einer ersten Knochenschraube (10) mit bidirektionaler Bohrspitze an einer zweiten Knochenschraube (10) mit bidirektionaler Bohrspitze zusammenzuwirken.

15. Bidirektionale Knochenschraube (10) mit Bohrspitze nach Anspruch 13, wobei die polyaxiale Verbindungsanordnung (18) konstruiert und angeordnet ist, um mit einem Plattenelement (58) zum Befestigen einer ersten Knochenschraube (10) mit bidirektionaler Bohrspitze an einer zweiten Knochenschraube (10) mit bidirektionaler Bohrspitze zusammenzuwirken.

## Revendications

1. Vis à os à pointe de forage bidirectionnelle (10) comprenant ;
- une tige (12) incluant un axe longitudinal central (13), une extrémité proximale (31) et une extrémité distale (15), au moins un fil hélicoïdal (28) s'étendant autour et le long d'une partie d'une surface extérieure (53) de la tige (12),
- au moins un fil hélicoïdal (28) adapté à la coopération par emboîtement avec un os,
- l'extrémité proximale (31) de la tige (12) incluant une pointe de forage bidirectionnelle (30),
- la pointe de forage bidirectionnelle (30) ayant au moins deux éléments d'âme (32) séparés par des gorges ouvertes (34),
- chaque composant d'âme (32) incluant une face de coupe (50) à l'extrémité la plus proximale de la tige (12), **caractérisée en ce que** chaque face de coupe (50) inclut au moins deux bords de coupe (40) agencés pour tourner autour de l'axe longitudinal central (13) et conçus pour couper lorsque la vis est soumise à une oscillation de rotation autour de l'axe longitudinal (13) de la tige (12),
- dans laquelle au moins un bord de coupe (40) des deux bords de coupe (40) est agencé pour couper lorsque la vis à os à pointe de forage bidirectionnelle (10) est tournée dans le sens des aiguilles d'une montre,
- et au moins un autre bord de coupe (40) des deux bords de coupe (40) est agencé pour couper lorsque la vis à os à pointe bidirectionnelle (10) est tournée dans le sens inverse des aiguilles d'une montre, l'oscillation rotative de la vis à os à pointe bidirectionnelle (10) autour de l'axe longitudinal (13) de la tige (12) étant appropriée pour former une ouverture d'un diamètre prédéterminé dans un os.

2. Vis à os à pointe bidirectionnelle (10) selon la revendication 1, dans laquelle la face de coupe (50) est plane et il y a un bord de coupe (40) de chaque côté de chaque gorge (34).

3. Vis à os à pointe bidirectionnelle (10) selon la revendication 1, dans laquelle chaque bord de coupe (40) inclut une inclinaison de face (42), l'inclinaison de face (42) étant agencée perpendiculairement par rapport à la face de coupe (50) s'étendant le long d'un composant d'âme respectif (32), et dans laquelle, de préférence, chaque bord de coupe (40) inclut une inclinaison de face (42), l'angle de la face (42) est inférieur à quatre-vingt-dix degrés par rapport à la face de coupe (50) qui s'étend le long d'un élément d'âme respectif (32), et dans lequel, de préférence, chaque bord de coupe (40) inclut un angle de la face (42), l'angle de la face (42) étant supérieur à quatre-vingt-dix degrés par rapport à la face de coupe (50) qui s'étend le long d'un élément d'âme respectif (32).

4. Vis à os à pointe bidirectionnelle (10) selon l'une quelconque des revendications précédentes, dans laquelle la pointe bidirectionnelle inclut au moins trois composants d'âme (32) séparés par des gorges ouvertes (34), chaque composant d'âme incluant une face de coupe (50) à l'extrémité la plus proximale de la tige (12), chaque face de coupe (50) incluant au moins un bord de coupe (40) agencé pour tourner autour de l'axe longitudinal central (13), dans laquelle au moins un bord de coupe (40) est agencé pour couper lorsque la vis à os à pointe de forage bidirectionnelle (10) est tournée dans le sens des aiguilles d'une montre et au moins un bord de coupe (40) est agencé pour couper lorsque la vis à os à pointe de forage bidirectionnelle (10) est tournée dans le sens inverse des aiguilles d'une montre.

5. Vis à os à pointe bidirectionnelle (10) selon l'une quelconque des revendications précédentes, dans laquelle la pointe bidirectionnelle comprend au moins quatre éléments d'âme (32) séparés par des gorges ouvertes (34), chaque élément d'âme (32) comprenant une face de coupe (50) à l'extrémité la plus proximale de la tige (12), chaque face de coupe (50) comprend au moins un bord de coupe (40) agencé pour tourner autour de l'axe longitudinal central (13), dans lequel au moins deux bords de coupes (40) sont agencés pour couper lorsque la vis à os à pointe de forage bidirectionnelle (10) est tournée dans le sens des aiguilles d'une montre et au moins deux bords de coupes (40) sont agencés pour couper lorsque la vis à os à pointe de forage bidirectionnelle (10) est tournée dans le sens inverse des aiguilles d'une montre.

6. Vis à os à pointe bidirectionnelle (10) selon l'une quelconque des revendications précédentes, dans laquelle chaque composant de l'âme (32) comprend une racine arrondie (44) et une âme (36), chaque âme (36) se terminant à la périphérie extérieure par un terrain (38), le terrain (38) étant construit et agencé pour contrôler le diamètre de l'ouverture créée par la vis à os à pointe bidirectionnelle (10).

7. Vis à os bidirectionnelle à pointe forée (10) selon la revendication 6, dans laquelle la surface (38) est construite et agencée pour brunir la surface de l'os lorsque l'ouverture est dimensionnée, et dans laquelle, de préférence, la surface (38) est construite et agencée pour comprimer la surface de l'os lorsque l'ouverture est dimensionnée.

8. Vis à os à pointe de forage bidirectionnelle (10) selon l'une quelconque des revendications précédentes, dans laquelle chaque face de coupe (50) est une surface plane.

9. Vis à os à pointe bidirectionnelle (10) selon la revendication 8, dans laquelle chaque face de coupe (50) est agencée à un angle par rapport à l'axe longitudinal (13) pour créer un angle de pointe (52), l'angle de pointe (52) réduisant la pression longitudinale nécessaire pour amorcer une ouverture.

10. Vis à os à pointe bidirectionnelle (10) selon l'une des revendications précédentes, dans laquelle les gorges (34) sont dimensionnées et formées pour canaliser les fragments et les copeaux d'os loin de l'extrémité proximale (31) lorsque la vis à os à pointe bidirectionnelle (10) est mis en oscillation.

11. Vis à os à pointe bidirectionnelle (10) selon l'une des revendications précédentes, dans laquelle au moins un filet hélicoïdal (28) est adapté pour couper des filets dans l'ouverture lorsque la vis à os à pointe bidirectionnelle (10) est tournée dans l'ouverture et dans laquelle, de préférence, au moins un filet hélicoïdal (28) est adapté pour former des filets par compression dans l'ouverture lorsque la vis à os à pointe bidirectionnelle (10) est tournée dans l'ouverture.

12. Vis à os à pointe bidirectionnelle (10) selon l'une quelconque des revendications précédentes, dans laquelle l'extrémité distale (15) de la tige (12) inclut une prise d'outil (17) pour coopérer avec un outil d'entraînement (14) pour la rotation et/ou l'oscillation de la tige (12), et dans laquelle, de préférence, l'extrémité distale (15) de la tige (12) inclut une partie d'une bille sphérique (16), la partie de la bille sphérique (16) étant formée d'un seul tenant avec la tige (12).

13. Vis à os à pointe bidirectionnelle (10) selon la revendication 12 incluant un assemblage de connecteur polyaxial (18), l'assemblage de connecteur polyaxial (18) incluant une douille (21) pour recevoir la partie d'une bille sphérique (16), la partie de la bille sphérique (16) et l'assemblage de connecteur polyaxial (18) coopérant pour permettre à l'axe longitudinal du connecteur (54) et à la tige (12) d'être positionnés à des angles l'un par rapport à l'autre.

14. Vis à os à pointe de forage bidirectionnelle (10) selon la revendication 13, dans laquelle l'assemblage de connecteur polyaxial (18) est construit et agencé pour coopérer avec un élément de tige (56) afin de fixer une première vis à os à pointe de forage bidirectionnelle (10) à une deuxième vis à os à pointe de forage bidirectionnelle (10).

15. Vis à os à pointe de forage bidirectionnelle (10) selon la revendication 13, dans laquelle l'assemblage de connecteur polyaxial (18) est construit et agencé pour coopérer avec un élément de plaque (58) afin de fixer une première vis à os à pointe de forage bidirectionnelle (10) à une deuxième vis à os à pointe de forage bidirectionnelle (10).
